# EUROPEAN PATENT SPECIFICATION

(11) **EP 0 374 692 B1**
(45) Date of publication and mention of the grant of the patent: **07.06.1995**
(21) Application number: 89122900.7
(22) Date of filing: 12.12.1989
(51) Int. Cl.: C07C 37/88

(54) **Use of hydroxycarboxylic acids as stabilizers for bisphenols**
Verwendung von Hydroxycarbonsäuren zur Stabilisation von Bisphenolen
Emploi d'acides hydroxycarboxyliques comme stabilisateurs de bisphénols

(30) Priority: 22.12.1988 US 289083
(43) Date of publication of application: 27.06.1990
(73) Proprietor: THE DOW CHEMICAL COMPANY, Midland Michigan 48640-1967 (US)
(72) Inventor: Neil, Lawrence E., Jr., Angleton Texas 77515 (US); Gormanos, Theodore J., Lake Jackson Texas 77566 (US)
(74) Representative: Sternagel, Hans-Günther, Dr.

(56) References cited:
- GB-A- 1 022 583
- US-A- 3 855 150
- US-A- 4 308 406
- US-A- 4 359 590
- English translation of JP-B-47043937

## Description

Most polymers are generally thought to be heat and oxygen sensitive and tend to degrade to form degradation products which have an adverse effect on the color of the polymers, as well as the physical properties of the polymers. Bisphenols, which are important in the preparation of epoxy resins and polyesters, are also known to be heat and oxygen sensitive, and when subjected to heat and/or oxygen, bisphenols form degradation products which adversely affect polymers subsequently made from the bisphenols containing these degradation products. Thus, bisphenol A is known to decompose with heat to form degradation products such as phenol and p-isopropylidene phenol. Additional heating of bisphenol A can cause formation of other degradation products such as complex non-volatile compounds. These degradation products from bisphenol A, even in small concentrations, can cause a lowering of the molecular weight of polyesters prepared from bisphenol A. Some of the degradation products formed in bisphenol can cause undesirable color in the bisphenol itself during is purification and in epoxy resins and/or polyesters since both purification and manufacturing processes employing bisphenol involve heating.

Stabilization of polymers from thermal degradation is believed to be achieved by incorporating a primary radical scavenger, usually a phenolic derivative. To combat the oxidation of the polymer one also must use a hydroperoxide decomposer together with the radical scavenger. Representative of hydroperoxide decomposers are thioesters and phosphites, such as dilaurylthiodipropionate and tri(nonylphenyl)phosphite. One reference, J. Appld. Polym. Sci., Vol. 27, 951-955 (1982), reports the investigation of tetrakis(2,4-di-tert-butylphenyl)4,4'-biphenylylene diphosphonite (SANDOSTAB PEPQ, a trademark of Sandoz Corporation) as both a hydroperoxide decomposer and a primary radical scavenger.

The presence of metal ions in bisphenol is also thought to have an adverse affect on the color of bisphenols, probably by promoting degradation. Various other additives have been employed to inhibit the formation of degradation products in bisphenols. Thus, alkaline earth phosphates, stannous oxide and oxalate, tin powder and tin dioxide, terephthalic and isophthalic acids, oxalic, sebacic and adipic acids and boron and antimony trioxides and their mixtures are useful additives for providing thermal stability to bisphenols as taught in GB-B-890,432.

GB-B-1,022,583, teaches that improved color stability is provided to bisphenols by incorporating oxalic, citric or tartaric acids or their alkali metal or ammonium salts in bisphenols during the bisphenol manufacturing process. It also teaches that the acids themselves or their ammonium salts are preferred and the acids or salts thereof may be added to the reactants for making bisphenol or to the reaction mixture after the reaction is complete, but before the bisphenol is separated from the reaction mixture.

US-A-3,629,339 teaches stabilizing phenols and bisphenols with an inorganic arsenic compound such as arsenic trioxide or ammonium and alkali metal arsenites.

US-A-4,160,110 teaches that various phthalic anhydrides are useful as distillation inhibitors against degradation of bisphenols. Thus, phthalic anhydride itself and tetrahydrophthalic anhydride are indicated by US-A-4,160,110 as useful as distillation inhibitors. JP-B-48-097854 discloses stabilizing bisphenol A by distilling it in the presence of a polypropylene glycol, epoxy soybean oil, 2,2-bis-(p-glycidylphenyl)-propane or a glycerol poly(oxypropylene) adduct.

Bisphenols are stabilized against thermal decomposition by incorporating therein a quaternary aliphatic ester of ortho titanic acid according to the teachings of US-A-4,359,590.

JP-B-47043937 teaches that the heat stability of bisphenol A can be improved by adjusting the pH of the Bisphenol A to between 2.0 and 5.0 by adding to the bisphenol A a weak acid such as glycolic, thioglycolic and polyphosphoric acid. JP-B-47043937 also reported testing other acids in addition to glycolic, thioglycolic and polyphosphonic acid for comparative purposes including phosphorous, boric and, lactic acid, none of which were considered to be effective. JP-B-47043937 reported that the use of lactic acid as an inhibitor produced no better results than an uninhibited product with respect to color when bisphenol A was tested for discoloration by heating in air for 1.5 hours at 250°C.

It is the object of the present invention to stabilize a bisphenol during its preparation or purification more effectively.

The object is solved by the use of lactic, malic, glyceric acids, or mixtures thereof, in form of the acids or as their ammonium or alkali metal salts or inhibiting the thermal degradation of a bisphenol, prepared from an aldehyde or a ketone and a phenol, during the preparation or purification of such bisphenol.

The present invention is based on the discovery that certain hydroxy carboxylic acids, not hitherto employed as stabilizers, act as excellent stabilizers (inhibitors). The hydroxy carboxylic acids of the present invention have been found to be excellent inhibitors against the degradation of bisphenols and especially for inhibiting the formation of degradation products which can cause color in the bisphenol itself or in the polymeric products made from bisphenol. The particular hydroxy carboxylic acids found to be useful as inhibitors are lactic, malic and glyceric, with lactic or malic being preferred. The acids of the present invention can also be employed as their ammonium or alkali metal salts. Lactic, malic and glyceric acids have been found to be superior to the hydroxy carboxylic acids heretofore employed.

The acids of the present invention are particularly useful in stabilizing bisphenols during purification of the bisphenols by distillation or during separation of the bisphenols from the reaction mixture, during which the bisphenols are exposed to elevated temperatures.

As is known in the art, bisphenols are prepared by reacting an aldehyde or a ketone with a phenol in the presence of an acid catalyst. Thus, for example. bisphenol A is produced by condensing phenol and acetone in the presence of an acid catalyst such as hydrochloric acid, sulfuric acid or a cation exchange resin in acid form and, if necessary, in the presence of a small amount of an accelerator such as a mercaptan Generally, bisphenol A is obtained from the reaction mixture by distilling or recrystallizing, extracting the residual bisphenol A directly from the reaction mixture or by distilling off excess phenol and water after neutralization.

Inhibitors or stabilizers employed by the prior art have been added to the feed reactants prior to the condensation reaction which forms the bisphenol. The prior art also teaches that inhibitors or stabilizers can be added at any point after the condensation reaction so long as the addition is prior to the distillation which separates the bisphenol from the reaction mixture. The hydroxy carboxylic acids of the present invention can be added to the feed reactants used to make the bisphenols or to the reaction mixture after the reaction is complete or at any point in between. While the inhibitor hydroxy carboxylic acids of the present invention can be added either to the reactants or in the reaction mixture, preferably the addition is carried out before the distillation of the bisphenol at which point degradation is most likely to occur. The inhibitors of the present invention perform well either in solution or in a melt containing the bisphenol.

The hydroxy carboxylic acids of this invention can be employed separately, in combination with each other, or in combination with other known inhibitors or stabilizers. The operable amount of the inhibitor of the present invention to be employed herein is in the range of from 2 ppm to 300 ppm based on the total weight of bisphenol. A preferred operable amount employed herein is from 5 ppm to 100 ppm and a most preferred operable amount employed herein is from 10 ppm to 30 ppm. Employing less than the above operable amount range will not satisfactorily inhibit the formation of degradation products in bisphenol. While employing more than 300 ppm herein will probably not be deleterious to the bisphenol, it is to no advantage.

### Test Apparatus and Method

A 4-neck, 2 L flask, containing a stirring device and thermometer, padded with nitrogen and fitted with a heating mantle was used to conduct the test for stabilizing bisphenols. Nitrogen pressure was maintained in the flask at 68.9 kPa (10 psig). A stainless steel mesh coupon was inserted through one of the necks of the flask and allowed to remain under the surface of liquid mixture contained in the flask for one hour while the flask was heated to a temperature of 80°C. The liquid mixture was continuously stirred throughout a two-hour period of heating. A 20-gram sample of the liquid mixture was removed from the flask at 1, 1½ and 2-hour intervals. Phenol and water was then stripped from the sample by a roto-evaporator at 180°C. Bisphenol was then checked for color using a Klett colorimeter. Klett readings taken by the Klett colorimeter were converted to APHA numbers based on ASTM (Pt-Co) P1209-69 standards.

The following experiments illustrate the inhibiting effect on the degradation of or the stabilization of the bisphenol by the compounds of the present invention:

### Example 1

To a reaction mixture containing water, acetone, phenol and bisphenol A was added 20 ppm lactic acid based on the bisphenol. The mixture was then heated to a temperature of 180°C and maintained thereat for two hours. The APHA color of the mixture was measured prior to the addition of the lactic acid and after the addition of the lactic acid from time to time during the two-hour period of heating. The increase in APHA color of a bisphenol mixture using a stabilizer of the present invention compared to that of an uninhibited bisphenol mixture (control) is shown in Table I.

### Examples 2 and 3

In the manner of Example 1, malic and glyceric acids were added to separate mixtures of water, acetone, phenol and bisphenol A (same basis). The APHA color of the mixture was measured prior to the addition of the acids and after the addition of the acids from time to time during the two-hour period of heating while the temperature of the mixture was maintained at 180°C. The increase in APHA color of the bisphenol mixture using the stabilizer of the present invention is shown in Table I along with a comparison of an uninhibited bisphenol mixture (control).

### Comparative Examples A, B, C and D

For comparative purposes, various compounds designated A, B, C and D in Table I were tested as inhibitors for bisphenol under the same conditions employed in Example 1. Results of the tests using inhibitors designated as comparative inhibitors A, B, C and D, Examples 1-3, and a control are shown in Table I.

**Table I**

| Stabilizer Compound | APHA Color at | | | |
|---|---|---|---|---|
| | 0 hr. | 1 hr. | 1½ hrs. | 2.0 hrs. |
| None (control) | 24 | 41 | 49 | 59 |
| Lactic acid | same | 30 | 33 | 41 |
| Malic acid | same | 27 | 29 | 32 |
| Glyceric acid | same | 22 | 26 | 29 |
| (A) Citric acid | same | 33 | 38 | 45 |
| (B) Tartaric Acid | same | 41 | 49 | 55 |
| (C) Glycolic Acid | same | 54 | 63 | 80 |
| (D) Oxalic Acid | same | 39 | 50 | 71 |

Other inhibitors known to the art and used commercially, including phosphoric acid, a tris(2,4-di-tert-butylphenyl)phosphite (IRGAFOS 168, a trademark of Ciba-Geigy Corporation) and SANDOSTAB PEPQ, were tested in the same manner as that of Example 1. The inhibitors of the present invention were shown to be superior to these commercially used inhibitors also.

## Claims

1. The use of lactic, malic, glyceric acids, or mixtures thereof, in form of the acids or as their ammonium or alkali metal salts for inhibiting the thermal degradation of a bisphenol, prepared from an aldehyde or a ketone and a phenol, during the preparation or purification of such bisphenol.

2. The use according to claim 1 wherein the acids are employed as their ammonium or alkali metal salts.

3. The use according to claim 1 wherein the bisphenol is bisphenol A.

4. The use according to claim 1 wherein the amount of the inhibitor is in the range of from 2 to 300 ppm based on the weight of the bisphenol.

5. The use according to claim 4 wherein the amount of the inhibitor is in the range of from 5 to 100 ppm.

6. The use according to claim 5 wherein the amount of the inhibitor is in the range of from 10 to 30 ppm.

## Patentansprüche

1. Verwendung von Milchsäure, Apfelsäure, Glycerinsäure oder Mischungen derselben in Form der Säuren oder ihrer Ammoniumsalze oder Alkalisalze zum Hemmen der thermischen Zersetzung eines Bisphenols, hergestellt aus einem Aldehyd oder einem Keton und einem Phenol, während der Herstellung oder Reinigung solchen Bisphenols.

2. Verwendung nach Anspruch 1,
**gekennzeichnet durch**
Verwendung der Säuren in Form ihrer Ammoniumsalze oder Alkalisalze.

3. Verwendung nach Anspruch 1,
**dadurch gekennzeichnet**,
daß das Bisphenol Bisphenol A ist.

4. Verwendung nach Anspruch 1,
**dadurch gekennzeichnet**,
daß die Menge des Inhibitors im Bereich von 2-300 ppm, bezogen auf Gewicht des Bisphenols, liegt.

5. Verwendung nach Anspruch 4,
**dadurch gekennzeichnet**,
daß die Menge des Inhibitors im Bereich von 5-100 ppm liegt.

6. Verwendung nach Anspruch 5,
**dadurch gekennzeichnet**,
daß die Menge des Inhibitors im Bereich von 10-30 ppm liegt.

## Revendications

1. Utilisation d'acides lactique, malique, glycérique, ou de mélanges de ceux-ci, sous forme d'acides ou de leurs sels d'ammonium ou de métaux alcalins, pour inhiber la dégradation thermique d'un bisphénol, préparé à partir d'un aldéhyde ou d'une cétone et d'un phénol, pendant la préparation ou la purification de ce bisphénol.

2. Utilisation selon la revendication 1, dans laquelle les acides sont utilisés sous forme de leurs sels d'ammonium ou de métaux alcalins.

3. Utilisation selon la revendication 1, dans laquelle le bisphénol est le bisphénol A.

4. Utilisation selon la revendication 1, dans laquelle la quantité de l'inhibiteur est comprise entre 2 et 300 ppm sur la base du poids du bisphénol.

5. Utilisation selon la revendication 4, dans laquelle la quantité de l'inhibiteur est comprise entre 5 et 100 ppm.

6. Utilisation selon la revendication 5, dans laquelle la quantité de l'inhibiteur est comprise entre 10 et 30 ppm.
